# EUROPÄISCHE PATENTSCHRIFT

(11) **EP 3 069 789 B1**
(45) Veröffentlichungstag und Bekanntmachung des Hinweises auf die Patenterteilung: **04.10.2017**
(21) Anmeldenummer: 15159843.0
(22) Anmeldetag: 19.03.2015
(51) Int. Cl.: B01J 39/20, B01J 47/02, B01J 47/026, C07C 255/46, C07C 253/10, C07C 253/34, B01J 31/02, B01J 31/40

(54) **VERFAHREN ZUR ABTRENNUNG VON KATIONEN AUS EINEM ISOPHORONNITRIL-PRODUKTGEMISCH**
METHOD FOR REMOVING CATIONS FROM AN ISOPHORONE NITRILE PRODUCT MIXTURE
PROCÉDÉ DE SÉPARATION DE CATIONS DANS UN MÉLANGE DE PRODUITS DE NITRILE D'ISOPHORONE

(43) Veröffentlichungstag der Anmeldung: 21.09.2016
(73) Patentinhaber: Evonik Degussa GmbH, 45128 Essen (DE)
(72) Erfinder: Nitz, Jörg-Joachim, 45257 Essen (DE); Kohlstruk, Stephan, 45966 Gladbeck (DE); Jansen, Robert, 46244 Bottrop (DE); Merkel, Andreas, 45665 Recklinghausen (DE); Müller, Anja, 44135 Dortmund (DE); Caßens, Jan, 45665 Recklinghausen (DE); Hengstermann, Axel, 48308 Senden (DE)

(56) Entgegenhaltungen:
- CN-A- 104 230 756
- DE-A1- 19 758 296

## Beschreibung

Die vorliegende Erfindungsmeldung betrifft die Abtrennung von Kationen aus einem bei der Isophoronnitrilproduktion anfallenden Stoffstrom mithilfe eines Kationenaustauschers.

Die Basen katalysierte Umsetzung von Blausäure (HCN) mit alpha, beta-ungesättigten cyclischen (oder acyclischen) Ketonen ist eine bekannte Reaktion (Hydrocyanation of Conjugated Carbonyl Compounds, CHAPTER 3, Wataru Nagata and Mitsuru Yoshioka, Wiley 2005).

Aus der EP 2 721 002 sind zahlreiche Verfahren zur Herstellung von Isophoronnitril bekannt, siehe die dort zitierten Dokumente.

In der EP 2 649 043 wird ein Verfahren zur Herstellung von Isophoronnitril beschrieben. Zur Abtrennung des Katalysators werden folgende Dokumente genannt:

Gemäß JP 06065182 kann nach Durchführung der Reaktion von HCN mit IP der alkalische Katalysator neutralisiert und das Reaktionsgemisch direkt in der Destillation aufgearbeitet werden. Alternativ kann gemäß JP 06065183 das alkalische Reaktionsgemisch mit einem inerten Lösemittel vermischt und in einem Dünnschichtverdampfer aufgearbeitet werden. Dabei werden der Katalysator, die Hochsieder und das Lösemittel abgetrennt. Das erhaltene Isophoronnitril wird dann in einer weiteren Kolonne aufgereinigt.

Die Abtrennung des Katalysators kann beispielsweise nach den in DE 10 85 871 oder EP 433 615 beschrieben Verfahren erfolgen.

Aus DE 197 58 296 A1 ist bekannt, Kationen aus einem Produktgemisch aus der Herstellung von Polytetrahydrofuran (PTHF) in Gegenwart eines Natrium-Kationen enthaltenen Katalysators in homogener Lösung (methanolische, natriumhaltige PTHF-Lösung) abzutrennen, wobei die Abtrennung der Natrium-Kationen des Katalysators mittels Kationenaustauscher ohne vorherige Neutralisation des Katalysators erfolgt.

Isophoronnitril (IPN) entsteht durch die Reaktion von Isophoron (IP) mit Blausäure unter Zuhilfenahme eines Katalysators. Vorzugsweise wird eine homogene Basenkatalyse eingesetzt, hierzu werden Alkalialkoholate, insbesondere Natriummethanolat, als Katalysator eingesetzt, wie in der EP 2649043 beschrieben.

CN 104230756 A offenbart ein Lithium- und/oder Natrium-Kationen enthaltendes Isophoronnitril-Produktgemisch aus der Herstellung von Isophoronnitril aus Isophoron und Blausäure mittels eines Kationen enthaltenden Katalysators. Die Abtrennung der Kationen dieses Produktgemisches erfolgt in einem zweistufigen Prozess mittels eines Kationenaustauschers, wobei eine vorherige Neutralisation des Katalysators erfolgt. Aufgabe war es, die Aufarbeitung des entstandenen Reaktionsgemisches aus der Herstellung von Isophoronnitril aus Isophoron und Blausäure zu verbessern.

Überraschenderweise wurde gefunden, dass sich durch die Verwendung eines Kationenaustauschers die Kationen des eingesetzten Katalysators entfernen lassen und die im Prozess verwendete Menge an Wasser stark reduzieren lässt wobei sich dadurch die nachgeschaltete Aufreinigung des Isophoronnitrils stark vereinfacht. Ebenfalls entfällt der Verfahrensschritt, dass der alkalische Katalysator vor seiner Abtrennung neutralisiert werden Gegenstand der Erfindung ist ein Verfahren zur Abtrennung von Kationen aus einem Isophoronnitril-Produktgemisch aus der Herstellung von Isophoronnitril aus Isophoron und Blausäure in Gegenwart eines Kationen enthaltenen Katalysators in homogener Phase, dadurch gekennzeichnet, dass
die Abtrennung der Kationen des Katalysators mittels Ionenaustauscher ohne vorherige Neutralisation des Katalysators erfolgt.

Die Abtrennung der Kationen erfolgt in einer lonentauscher-Apparatur, die sowohl ein- oder mehrstufig ausgelegt sein kann. Bevorzugt erfolgt die Abtrennung einstufig in nur einer Ionenaustauscher Apparatur.

Zur Herstellung von Isophoronnitril wird Isophoron und Blausäure umgesetzt, wobei die Reaktion in Gegenwart eines kationischen Katalysators durchgeführt wird.

Die Umsetzung wird homogen basenkatalysiert durchgeführt. Bei der homogenen Katalyse liegen Katalysator und Reaktanden in der gleichen Phase vor, also in homogener Phase. Bevorzugt handelt es sich bei dem Katalysator um ein in einem Lösungsmittel gelöstes Metallsalz, an dem die Reaktanden zum Produkt Isophoronnitril reagieren.

Als Katalysatoren werden in dem erfindungsgemäßen Verfahren vorzugsweise lösliche Katalysatoren aus der Gruppe der basischen Alkali- oder Erdalkalimetallverbindungen eingesetzt. Hierzu werden bevorzugt Alkalialkoholate, insbesondere Natriummethanolat, als Katalysatoren eingesetzt.

Bevorzugt wird erfindungsgemäß eine 20 - 35 Gew.-%ige Natriummethanolatlösung, besonders bevorzugt eine 25 - 30 Gew.-%ige Natriummethanolatlösung, gelöst in Methanol eingesetzt.

Die eingesetzte Blausäure kann in reiner Form aber auch als Gemisch mit Stabilisatoren eingesetzt werden. Als Stabilisatoren eignen sich alle dem Fachmann für diesen Zweck bekannten Verbindungen, beispielsweise Phosphorsäure, Schwefeldioxid und Oxalsäure.

Bevorzugt werden Phosphorsäure, Schwefeldioxid oder Gemische hieraus als Stabilisatoren für die Blausäure eingesetzt. Der Anteil der Stabilisatoren liegt üblicherweise im Bereich von 0,1 bis 2 Gew.-%, bezogen auf die eingesetzte Blausäure, vorzugsweise im Bereich von 0,5 bis 1 Gew.-%.

Die Umsetzung kann in Gegenwart oder Abwesenheit von inerten Lösungsmitteln vorgenommen werden. Besonders bevorzugt verwendet man Isophoron im molaren Überschuss, bezogen auf die eingesetzte Blausäure und setzt kein externes Lösungsmittel zu.

Generell wird das Verfahren derart durchgeführt, dass ein Überschuss an Isophoron eingesetzt wird, da so eine höhere Selektivität zu Isophoronnitril erzielt wird. Im Allgemeinen beträgt das molare Verhältnis Isophoron/HCN >1:1, in der Regel 19:1 bis 1,5:1, vorzugsweise 3:1 bis 1,5:1.

Die Katalysatorkonzentration, bezogen auf die eingesetzte Isophoronmenge, liegt im Bereich von 0,03 bis 20 Gew.-%, vorzugsweise im Bereich von 0,03 bis 1 Gew.-% .

Die Gesamtmenge des Isophorons kann vorgelegt und auf die gewünschte Reaktionstemperatur gebracht werden, bevor in Anwesenheit des Katalysators das HCN zugegeben wird.

Die HCN Zudosierung erfolgt vorzugsweise so, dass eine homogene Verteilung der Edukte (Isophoron und HCN) gewährleistet wird. Die HCN Zudosierung geschieht auf übliche, dem Fachmann bekannte Weise, z. B. über Statikmischer, Pumpen oder Verdüsung.

Die Reaktionstemperaturen liegen üblicherweise im Bereich von 130 bis 225 °C, bevorzugt im Bereich von 135 bis 195 °C und ganz besonders bevorzugt im Bereich von 140 bis 175 °C.

Die Reaktion wird bei Drücken von 0,01 bis 10 bar, bevorzugt 1 bis 5 bar, besonders bevorzugt bei Normaldruck (Atmosphärendruck) durchgeführt.

Innerhalb der vorstehend dargelegten Reaktionsparameter wird das HCN so zudosiert, dass eine ausreichend niedrige Konzentration von HCN resultiert und somit eine hohe Selektivität sowie ein hoher Umsatz zu Isophoronnitril erzielt werden kann. Die Selektivität hinsichtlich IPN sollte > 95 %, bevorzugt > 98 %, besonders bevorzugt > 99 %, liegen.

Dabei darf nur eine geringe Polymerisation des HCN auftreten, da hierdurch der Umsatz und die Selektivität negativ beeinflusst würden.

Vorzugsweise werden die Konzentrationen an nicht umgesetztem, freiem HCN und die Gesamtkonzentration an Cyanidionen (Summe aus freiem HCN und als Cyanhydrine von Isophoron und Isophoronnitril gebundenem Cyanid) bestimmt und die Reaktionsbedingungen so angepasst, dass eine optimierte Selektivität hinsichtlich IPN erhalten wird. Die Bestimmung der genannten Cyanidionen-Konzentrationen erfolgt vorzugsweise durch Titration.

Das Verfahren kann in bevorzugter Weise kontinuierlich, diskontinuierlich oder semikontinuierlich durchgeführt werden, insbesondere kontinuierlich.

In bevorzugter Ausführung wird die Reaktionsdurchführung, je nach eingesetztem Katalysator, in einem Rührkessel, einer Rührkesselkaskade, einem Kreislaufreaktor, einem Strömungsrohr, einem oder mehreren Festbettreaktoren oder einer Kolonne durchgeführt.

Die Aufarbeitung des Reaktionsgemisches, welches nach beendeter Reaktion im Wesentlichen das Endprodukt Isophoronnitril enthält, aber auch noch Anteile an Edukten wie Isophoron, HCN und auch Lösemittel und Nebenprodukte enthalten kann, und den Katalysator enthält, erfolgt erfindungsgemäß zuerst durch die Abtrennung der Kationen aus dem Katalysator an einem Kationenaustauscher ohne vorherige Neutralisation des Katalysators.

Prinzipiell sind alle Kationenaustauscher einsetzbar. Besonders bevorzugt sind immobilisierte lonentauscher die als funktionelle Gruppen Sulfonsäuregruppen aufweisen, die auf eine mit Divinylbenzol quervernetzte Polystyrolmatrix aufgebracht wurden.

Die Abtrennung der Kationen des Katalysators ohne dessen Neutralisation, insbesondere des bevorzugten Natriummethanolats, erfolgt als erster Aufreinigungsschritt nach dem Reaktor. Dabei wird das Reaktionsprodukt über in einer oder mehreren Adsorptionskolonnen immobilisierte Kationenaustauscher gefahren. Die im Reaktionsgemisch enthaltenen Kationen, insbesondere die Natriumionen, werden gegen Wasserstoffionen des Ionenaustauschers ausgetauscht. Dadurch wird das Kation, insbesondere Natrium aus dem Produktstrom entfernt.

Die Abtrennung der Kationen erfolgt dabei ohne die Zugabe weiterer chemischer Zusatzstoffe.

Erfindungsgemäß bevorzugt wird als Katalysator Natriummethanolat gelöst in Methanol eingesetzt, wobei das Methanolat zu Methanol umgewandelt indem die Natriumionen gegen Wasserstoffionen des Ionenaustauschers ausgetauscht werden. Der so entstehende Produktstrom enthält im Vergleich zum üblichen Prozess weniger Wasser, bevorzugt kein Wasser, wodurch die weiteren Aufreinigungsschritte vereinfacht werden.

Nach der Abtrennung der Kationen des Katalysators wird das erhaltene Stoffgemisch nach üblichen Verfahren weiter aufgearbeitet und anschließend des Isophoronnitril als Endprodukt isoliert.

Bevorzugt erfolgt die weitere Aufarbeitung des Stoffgemisches nach der lonenaustausch-Apparatur erfindungsgemäß nach den folgenden beschriebenen zwei Varianten.

Für die Aufreinigung des bei der Neutralisation eingesetzten Wassers gemäß des Stands der Technik ist eine zusätzliche Kolonne notwendig. Bei dem Verfahren gemäß der Erfindung fällt diese Kolonne deutlich kleiner aus bzw. entfällt komplett. Dies begründet einen weiteren technischen Fortschritt.

Bei der ersten Variante erfolgt die Abtrennung der Leichtsieder, erfindungsgemäß bevorzugt Methanol, in einer dem Ionenaustauscher nachgeschalteten ersten Kolonne. Diese Kolonne kann im Vergleich zum Verfahren mit Neutralisation deutlich kleiner ausfallen, da die Menge der abzutrennenden Leichtsieder wesentlich reduziert wird. In einer zweiten nachgeschalteten Kolonne findet dann die Aufreinigung des Isophoronnitrils statt. Die Schwersieder verlassen die Kolonne am Sumpf, Isophoron geht über Kopf und das Isophoronnitril wird seitlich rein gewonnen.

In der zweiten Variante wird die Aufreinigung des Stoffgemisches nach der lonenaustausch-Apparatur in einer nachgeschalteten Kolonne, welche am Kopf aufgesetzt oder innerhalb der Kolonne einen Partialkondensator enthält, durchgeführt. Dabei werden die Abtrennung der Leichtsieder, Isophoron sowie die Schwersieder in nur einer dem Ionenaustauscher nachgeschalteten Kolonne kombiniert. Dazu wird der Kopfstrom in der Kolonne durch einen enthaltenen Partialkondensator nur teilweise kondensiert um das Isophoron von Leichtsiedern, erfindungsgemäß bevorzugt Methanol, getrennt. Die Schwersieder verlassen die Kolonne am Sumpf und das Isophoronnitril wird seitlich rein gewonnen.

### Beispiele

### 1.

Um die adsorptive Abtrennung von Natrium aus Rohisophoronnitril zu untersuchen wurden Versuche im Labormaßstab durchgeführt. Dazu wurde eine doppelwandige Glassäule mit einem Innendurchmesser von 20 mm verwendet. Diese wurde mit dem lonentauscher befüllt, das Rohisophoronnitril wurde über eine Pumpe auf 70°C vorgewärmt oben auf den lonentauscher aufgegeben, so dass das Isophoronnitril schwerkraftgetrieben durch die Säule gefördert wurde.

Für die Versuche wurde Rohisophoronnitril, im Wesentlichen enthaltend: Isophoronnitril, Isophoron, Schwersieder, Methanol, Natriummethanolat, aus einer Produktionsanlage verwendet. Es wurden folgende lonentauscher getestet:
- Lewatit K2629 (Lanxess)
- PPC 100H (Purolite)
- PPC 150H (Purolite)
- Amberlyst 15 WET (DOW)

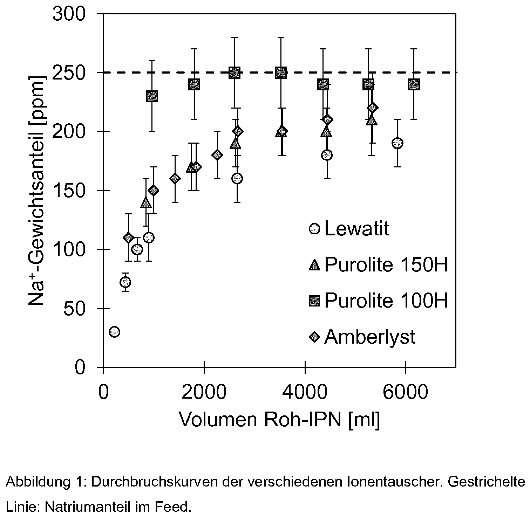

Alle ausgewählten lonentauscher besitzen als funktionelle Gruppen Sulfonsäuregruppen, die auf eine mit Divinylbenzol quervernetzte Polystyrolmatrix aufgebracht wurden. Abbildung 1 stellt die Durchbruchskurven des Natriums der verschiedenen lonentauscher für einen Rohisophoronnitril-Volumenstrom von 15 ml/min gegenüber. Hierbei ist der Natriumgewichtsanteil im Austritt aus dem lonentauscher über die Menge an verwendetem Rohisophoron aufgetragen. Neben den Durchbruchskurven ist der Natriumgewichtsanteil im Feed zum lonentauscher als gestrichelte Linie dargestellt. Bis zum Ende der Messungen konnte so auch nur etwa 25 % der aktiven Wasserstoffionen durch Natriumionen ausgetauscht werden. Die erste erfasste Natriumionenkonzentration lag nach nur 220 ml aufgereinigtem Isophoronnitril schon bei 30 ppm, hierbei waren allerdings nur ca. 3 % der Wasserstoffionen ausgetauscht.

### 2.

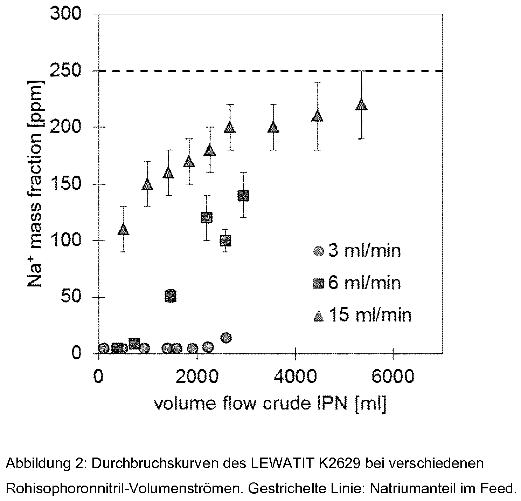

Dies wird auch durch die Variation des Volumenstroms bei ansonsten gleichen Bedingungen für den Kationentauscher Lewatit K2629 bestätigt. In Abbildung 2 sind die verschiedenen Volumenströme gegenübergestellt. Abbildung 3 ist zu entnehmen, dass sich die Durchbruchskurve hin zu größeren aufgereinigten Volumina verschiebt, je kleiner der Rohisophoronnitril-Volumenstrom ist.

## Patentansprüche

1. Verfahren zur Abtrennung von Kationen aus einem Isophoronnitril-Produktgemisch aus der Herstellung von Isophoronnitril aus Isophoron und Blausäure in Gegenwart eines Kationen enthaltenden Katalysators in homogener Phase, **dadurch gekennzeichnet, dass** die Abtrennung der Kationen des Katalysators mittels Kationenaustauscher ohne vorherige Neutralisation des Katalysators erfolgt.

2. Verfahren nach Anspruch 1, **dadurch gekennzeichnet, dass** die Abtrennung der Kationen in einer lonenaustauscher-Apparatur erfolgt, die einstufig oder mehrstufig ausgelegt ist.

3. Verfahren nach mindestens einem der vorherigen Ansprüche, **dadurch gekennzeichnet, dass** die Abtrennung der Kationen einstufig in nur einer Ionenaustauscher-Apparatur erfolgt.

4. Verfahren nach mindestens einem der vorherigen Ansprüche 2-3, **dadurch gekennzeichnet, dass** die weitere Aufarbeitung des Produktgemisches nach der Ionenaustauscher-Apparatur erfolgt durch Abtrennung der Leichtsieder in einer dem Ionenaustauscher nachgeschalteten ersten Kolonne, und anschließende Aufreinigung des Isophoronnitrils in einer zweiten nachgeschalteten Kolonne.

5. Verfahren nach mindestens einem der vorherigen Ansprüche 2-3 **dadurch gekennzeichnet, dass** die weitere Aufreinigung des Produktgemisches nach der lonenaustauscher-Apparatur in einer nachgeschalteten Kolonne, welche am Kopf aufgesetzt oder innerhalb der Kolonne einen Partialkondensator enthält, durchgeführt wird.

6. Verfahren nach mindestens einem der vorherigen Ansprüche, **dadurch gekennzeichnet, dass** immobilisierte Ionenaustauscher, die als funktionelle Gruppen Sulfonsäuregruppen aufweisen, die auf eine mit Divinylbenzol quervernetzte Polystyrolmatrix aufgebracht wurden, eingesetzt werden.

7. Verfahren nach mindestens einem der vorherigen Ansprüche, **dadurch gekennzeichnet, dass** als Katalysatoren lösliche Katalysatoren aus der Gruppe der basischen Alkali- oder Erdalkalimetallverbindungen eingesetzt werden.

8. Verfahren nach mindestens einem der vorherigen Ansprüche, **dadurch gekennzeichnet, dass** Alkalialkoholate, insbesondere Natriummethanolat, als Katalysatoren eingesetzt werden.

9. Verfahren nach mindestens einem der vorherigen Ansprüche, **dadurch gekennzeichnet, dass** eine 20-35 Gew.-%ige Natriummethanolatlösung, gelöst in Methanol eingesetzt wird.

## Claims

1. Process for removing cations from an isophoronenitrile product mixture from the preparation of isophoronenitrile from isophorone and hydrocyanic acid in the presence of a cation-containing catalyst in homogeneous phase, **characterized in that** the removal of the cations of the catalyst takes place by means of cation exchanger without prior neutralization of the catalyst.

2. Process according to Claim 1, **characterized in that** the removal of the cations takes place in an ion exchanger apparatus which is designed to be single-stage or multi-stage.

3. Process according to at least one of the preceding claims, **characterized in that** the removal of the cations takes place in a single-stage in only one ion exchanger apparatus.

4. Process according to at least one of the preceding Claims 2-3, **characterized in that** the further work-up of the product mixture takes place after the ion exchanger apparatus by
removal of the low boilers in a first column downstream of the ion exchanger,
and subsequent purification of the isophoronenitrile in a second downstream column.

5. Process according to at least one of the preceding Claims 2-3, **characterized in that** the further purification of the product mixture after the ion exchanger apparatus is carried out in a downstream column which contains a partial condenser placed on top or within the column.

6. Process according to at least one of the preceding claims, **characterized in that** immobilized ion exchangers which have, as functional groups, sulfonic acid groups which have been applied to a polystyrene matrix crosslinked with divinylbenzene are used.

7. Process according to at least one of the preceding claims, **characterized in that** the catalysts used are soluble catalysts from the group of basic alkali metal or alkaline earth metal compounds.

8. Process according to at least one of the preceding claims, **characterized in that** alkali metal alcoholates, in particular sodium methanolate, are used as catalysts.

9. Process according to at least one of the preceding claims, **characterized in that** a 20-35% by weight sodium methanolate solution, dissolved in methanol is used.

## Revendications

1. Procédé pour la séparation de cations d'un mélange de produits à base d'isophoronenitrile provenant de la préparation d'isophoronenitrile à partir d'isophorone et d'acide cyanhydrique en présence d'un catalyseur contenant des cations en phase homogène, **caractérisé en ce que** la séparation des cations du catalyseur a lieu au moyen d'échangeur cationique sans neutralisation préalable du catalyseur.

2. Procédé selon la revendication 1, **caractérisé en ce que** la séparation des cations a lieu dans un appareil d'échange ionique qui est conçu à un ou plusieurs étages.

3. Procédé selon au moins l'une quelconque des revendications précédentes, **caractérisé en ce que** la séparation des cations a lieu en un étage dans seulement un appareil d'échange ionique.

4. Procédé selon au moins l'une quelconque des revendications précédentes 2-3, **caractérisé en ce que** le traitement ultérieur du mélange de produits après l'appareil d'échange ionique a lieu par séparation de la fraction légère dans une première colonne disposée en aval de l'échangeur d'ions et par purification consécutive de l'isophoronenitrile dans une deuxième colonne disposée en aval.

5. Procédé selon au moins l'une quelconque des revendications précédentes 2-3, **caractérisé en ce que** la purification ultérieure du mélange de produits après l'appareil d'échange ionique est réalisée dans une colonne disposée en aval qui contient un condenseur partiel placé sur la tête ou dans la colonne.

6. Procédé selon au moins l'une quelconque des revendications précédentes, **caractérisé en ce que** des échangeurs d'ions immobilisés, qui présentent des groupes acide sulfonique comme groupes fonctionnels, qui ont été appliqués sur une matrice de polystyrène réticulée par du divinylbenzène, sont utilisés.

7. Procédé selon au moins l'une quelconque des revendications précédentes, **caractérisé en ce qu'**on utilise, comme catalyseurs, des catalyseurs solubles du groupe des composés basiques à base de métal alcalin ou de métal alcalino-terreux.

8. Procédé selon au moins l'une quelconque des revendications précédentes, **caractérisé en ce qu'**on utilise des alcoolates de métal alcalin, en particulier du méthanolate de sodium, comme catalyseurs.

9. Procédé selon au moins l'une quelconque des revendications précédentes, **caractérisé en ce qu'**on utilise une solution de méthanolate de sodium à 20-35% en poids, dissous dans du méthanol.
